# EUROPÄISCHE PATENTSCHRIFT

(11) **EP 1 477 122 B1**
(45) Veröffentlichungstag und Bekanntmachung des Hinweises auf die Patenterteilung: **12.11.2008**
(21) Anmeldenummer: 04015445.2
(22) Anmeldetag: 23.05.2002
(51) Int. Cl.: A61B 17/68

(54) **Implantat zur Fixierung von Knochenplatten**
Implant for fixing bone plates
Implant destiné à la fixation de plaques d'osteosynthése

(30) Priorität: 15.06.2001 DE 10128917
(43) Veröffentlichungstag der Anmeldung: 17.11.2004
(62) Teilanmeldung aus: 02743080.0
(73) Patentinhaber: Aesculap AG, 78532 Tuttlingen (DE)
(72) Erfinder: Nesper, Markus, 78532 Tuttlingen (DE); Steinhilper, Klaus-Dieter, 78532 Tuttlingen (DE); Weisshaupt, Dieter, 78194 Immendingen (DE)
(74) Vertreter: Regelmann, Thomas

(56) Entgegenhaltungen:
- DE-U- 29 919 090
- US-A- 5 021 059
- US-A- 5 921 986

## Beschreibung

Die Erfindung betrifft ein Implantat zur Fixierung benachbarter, eine Innenseite und eine Außenseite aufweisender Knochenplatten des Schädelknochens mit einem einen Trennspalt zwischen den Knochenplatten überdeckenden inneren Anlageelement, einem den Trennspalt überdeckenden äußeren Anlageelement und mit den Trennspalt durchsetzenden Verbindungsmitteln.

Implantate dieser Art sind beispielsweise aus dem deutschen Gebrauchsmuster 29919090 bekannt. Sie werden verwendet, um im Bereich des Schädelknochens aus diesem durch einen Sägeschnitt herausgetrennte Knochenplatten wieder in der ursprünglichen Position festzulegen, so daß sie in dieser Position einheilen können.

Zu diesem Zweck ist es bekannt, tellerförmige Anlageelemente auf beiden Seiten der nur durch einen Trennspalt voneinander getrennten Knochenplatten anzuordnen und diese durch einen zentralen Stift oder ein zentrales Rastband miteinander zu verbinden. Es ist außerdem bekannt, statt derartiger Rast-oder Klemm-Mittel die beiden tellerförmigen Anlageelemente einfach durch ein fadenförmiges Spannelement gegeneinander zu spannen und dann durch dieses fadenförmige Spannelement gegeneinander gespannt zu halten.

Bei der Verwendung von stiftförmigen Verbindungsmitteln ist es notwendig, die beiden Anlageelemente durch ein geeignetes Werkzeug gegeneinander zu spannen, welche an dem Verbindungsmittel angreift, welches das äußere Anlageelement durchsetzt, und welches gleichzeitig das äußere Anlageelement längs des Verbindungsmittels in Richtung des inneren Anlageelementes verschiebt. Derartige Instrumente sind kompliziert aufgebaut und nicht immer einfach zu handhaben.

Es ist Aufgabe der Erfindung, ein gattungsgemäßes Implantat derart auszubilden, daß das Anlegen der Anlageelemente und das Spannen der Anlageelemente möglichst einfach ausführbar ist.

Diese Aufgabe wird bei einem Implantat der eingangs beschriebenen Art erfindungsgemäß dadurch gelöst, daß die beiden Anlageelemente durch ein fadenförmiges Spannelement verbunden sind, welches durch das äußere Anlageelement verschieblich hindurchgeführt ist und bei Zug das innere Anlageelement gegen das äußere Anlageelement verschiebt, daß das Spannelement durch zwei Öffnungen im inneren Anlageelement hindurchgeführt ist und im Bereich zwischen den Öffnungen auf der dem äußeren Anlageelement abgewandten Außenseite des inneren Anlageelementes verläuft und daß das Spannelement von der Außenseite des inneren Anlageelementes durch dieses hindurch in einer Schlaufe geführt ist, die durch zwei im Abstand zueinander angeordnete Öffnungen in dem äußeren Anlageelement verläuft, so daß die Schlaufe zwischen diesen Öffnungen an der vom inneren Anlageelement abgewandten Außenseite des äußeren Anlageelementes anliegt, wobei die Schlaufe durch zwei im Abstand zueinander angeordnete Öffnungen am inneren Anlageelement hindurchläuft.

Es werden also an dem Implantat fadenförmige Spannelemente vorgesehen, deren Aufgabe im wesentlichen darin besteht, beim Anlegevorgang die beiden Anlageelemente gegeneinander zu spannen.

Das fadenförmige Spannelement kann als Sicherung der beiden Anlageelemente verbleiben, beispielsweise können die Enden eines derartigen fadenförmigen Spannelementes miteinander verknotet werden.

Es ist vorgesehen, daß das Spannelement durch zwei Öffnungen im inneren Anlageelement hindurchgeführt ist und im Bereich zwischen den Öffnungen auf der dem äußeren Anlageelement abgewandten Außenseite des inneren Anlageelementes verläuft. Das Spannelement wird also in einer U-förmigen Schlaufe geführt, wobei die beiden Enden des Spannelementes durch Öffnungen im inneren Anlageelement hindurchgeführt sind und sich von dort durch den Trennspalt hindurch zum äußeren Anlageelement hin erstrecken. Dabei ist es günstig, wenn die beiden Enden des Spannelementes durch je eine Öffnung des äußeren Anlageelementes hindurchgeführt sind.

Es ist vorgesehen, daß das Spannelement von der Außenseite des inneren Anlageelementes durch dieses hindurch in einer Schlaufe geführt ist, die durch zwei im Abstand zueinander angeordnete Öffnungen in dem äußeren Anlageelement verläuft, so daß die Schlaufe zwischen diesen Öffnungen an der vom inneren Anlageelement abgewandten Außenseite des äußeren Anlageelementes anliegt. Man erhält durch diese Fadenführung zwischen den beiden Anlageelementen insgesamt vier Fadenabschnitte, es wird also eine Art Flaschenzug ausgebildet, so daß einer Verkürzung des fadenförmigen Spannelementes eine Verschiebung der Anlageelemente entspricht, die entsprechend untersetzt erfolgt, jedoch mit erhöhter Kraft. Dabei kann vorgesehen sein, daß die Öffnungen der Schlaufe und die Öffnungen der Enden des Spannelementes am äußeren Anlageelement auf einer Geraden liegen, insbesondere sind diese Öffnungen äquidistant zueinander angeordnet.

Es ist auch vorgeschen, daß die Schlaufe dabei durch zwei im Abstand zueinander angeordnete Öffnungen im inneren Anlageelement hindurchläuft. Diese Öffnungen können zusammen mit den Öffnungen der Enden des Spannelementes auf einer Geraden liegen und insbesondere äquidistant zueinander angeordnet sein.

Es ist günstig, wenn die beiden Öffnungen im inneren Anlageelement einen Abstand voneinander haben, der mindestens der halben Ausdehnung des inneren Anlageelementes in ihrer Verbindungsrichtung entspricht. Damit greifen die beiden Enden des Spannelementes an gegenüberliegenden Außenseiten des inneren Anlageelementes an, dabei richtet sich das Anlageelement zwangsläufig so aus, daß die Verbindungsrichtung der beiden Öffnungen im inneren Anlageelement für die beiden Enden des Spannelementes parallel zur Richtung des Trennspaltes verläuft.

Besonders vorteilhaft ist es, wenn Verbindungsmittel zwischen den beiden zwischen den Anlageelementen verlaufenden Abschnitten des Spannelementes angeordnet sind. Dadurch, daß die beiden Enden des Spannelementes im gegenseitigen Abstand zwischen den beiden Anlageelementen geführt sind, bleibt zwischen ihnen genügend Platz, dort die Verbindungsmittel anzuordnen, beispielsweise zentral.

Dabei ist es weiterhin vorteilhaft, wenn Verbindungsmittel in einer Richtung quer zu der von den zwischen den beiden Anlageelementen verlaufenden Abschnitten des Spannelementes aufgespannten Ebene eine geringe Ausdehnung aufweisen, um so den Durchgang durch den Trennspalt zu ermöglichen. Die Verbindungsmittel und die Spannmittel befinden sich also im wesentlichen in einer Ebene und erstrecken sich nur in dieser Ebene und möglichst wenig quer zu dieser Ebene, so daß die beschriebenen Elemente ohne weiteres auch durch einen sehr schmalen Trennspalt hindurchgeführt werden können, es ist also möglich, die miteinander zu verbindenden Knochenplatten relativ dicht aneinander heranzuführen.

Besonders günstig ist dabei eine Anordnung, wenn die zwischen den Anlageelementen verlaufenden Abschnitte des Spannelementes, die Schlaufe und Verbindungsmittel in einer Ebene angeordnet sind.

Auch ist es günstig, wenn das Spannelement aus einem im Körper resorbierbaren Material besteht.

Bei einer bevorzugten Ausführungsform der Erfindung ist vorgesehen, daß Verbindungsmittel im Bereich zwischen den Anlageelementen angeordnet sind und nicht durch diese hindurch nach außen ragen. Durch die Verwendung der flexiblen Spannmittel ist es möglich, die Anlageelemente auch dann zuverlässig gegeneinander zu spannen, wenn keine Verbindungsmittel durch das äußere Anlageelement nach außen hindurchragen.

Zum Beispiel können an beiden Anlageelementen an deren dem anderen Anlageelement zugewandten Innenseiten Rastvorsprünge angeordnet sein, die sich bei Annäherung der Anlageelemente rastend hintergreifen.

Vorteilhaft ist es dabei, wenn an einem Anlageelement nebeneinander zwei Rastelemente angeordnet sind, zwischen die ein Rastelement am anderen Anlageelement bei Annäherung der Anlageelemente rastend eingreift.

Eine stufenweise Verrastung und damit eine Anpassung an die Dicke der aneinander zu fixierenden Knochenplatten wird möglich, wenn mindestens eines der Rastelement eine Vielzahl von Rastvorsprüngen trägt.

Bei einer anderen bevorzugten Ausführungsform ist vorgesehen, daß nebeneinander an den Anlageelementen mindestens zwei Verbindungsmittel angeordnet sind, die quer zu der von ihnen definierten Ebene eine geringe Ausdehnung aufweisen.

Im Gegensatz zu bekannten Anlageelementen, die in der Regel ein zentrales Verbindungsmittel verwenden, werden also hier nebeneinander an den Anlageelementen mindestens zwei derartige Verbindungsmittel verwendet, die eine Ebene aufspannen, und diese Ebene geht bei angelegten Anlageelementen durch den Trennspalt hindurch. Durch die Verwendung von zwei Verbindungsmitteln wird das Anlageelement relativ zu den Knochenplatten so orientiert, daß diese Ebene parallel zum Trennspalt verläuft, es ist außerdem möglich, gegebenenfalls die Anlageelemente auch in einer nicht parallelen Anordnung festzulegen, falls dies durch unterschiedliche Dicken der zu verbindenden Knochenplatten notwendig ist.

Günstig ist es, wenn Verbindungsmittel Rast- oder Klemmstifte umfassen, die am inneren Anlageelement festgelegt sind und Rast- oder Klemmöffnungen in dem äußeren Anlageelement durchsetzen.

Diese Rast- oder Klemmöffnungen können Rastvorsprünge tragen, die mit Rastvorsprüngen an den Rast- oder Klemmstiften zusammenwirken.

Bei einer anderen Ausführungsform ist vorgesehen, daß die Rast- oder Klemmöffnungen elastisch verformbare Klemmglieder aufweisen, die mit der Oberfläche der Rast- oder Klemmstifte klemmend zusammenwirken. Beispielsweise können diese Klemmglieder federnde Zungen sein, die sich aus der Ebene der Anlageelemente herausbiegen, wenn die Rast- oder Klemmstifte durch die Rast- oder Klemmöffnungen verschoben werden, und die mit ihrer Kante an der Oberfläche der Rast- oder Klemmstifte so anliegen, daß eine Relativverschiebung der Rast- oder Klemmstifte gegenüber dem Anlageelemente nur in einer Richtung möglich ist.

Die Anlageelemente und Verbindungsmittel sind vorzugsweise einstückig aus Kunststoff hergestellt.

Die nachfolgende Beschreibung bevorzugter Ausführungsformen dient in Zusammenhang mit der Zeichnung der näheren Erläuterung. Es zeigen:
- Figur 1:: eine Seitenansicht eines Beispiels eines Implantates mit einem fadenförmigen Spannelement in U-Form und mit zum jeweils anderen Anlageelement weisenden Rastvorsprüngen vor dem Zusammenführen der Anlageelemente, wobei dieses Implantat nicht unter die Ansprüche fällt;
- Figur 2:: eine Schnittansicht längs Linie 2-2 in Figur 1;
- Figur 3:: eine Ansicht des Implantates der Figur 1 in Richtung des Pfeiles A in Figur 1 nach dem Anlegen des Implantates an zwei Knochenplatten;
- Figur 4:: eine Ansicht ähnlich Figur 1 bei einem weiteren bevorzugten Ausführungsbeispiel eines Implantates mit zwei im Abstand zueinander angeordneten Verbindungsmitteln und mit einem fadenförmigen Spannelement in Mehrfachschlaufenführung und
- Figur 5:: eine Ansicht des Implantates der Figur 4 in Richtung des Pfeiles B in Figur 4 nach dem Anlegen des Implantates an zwei Knochenplatten.

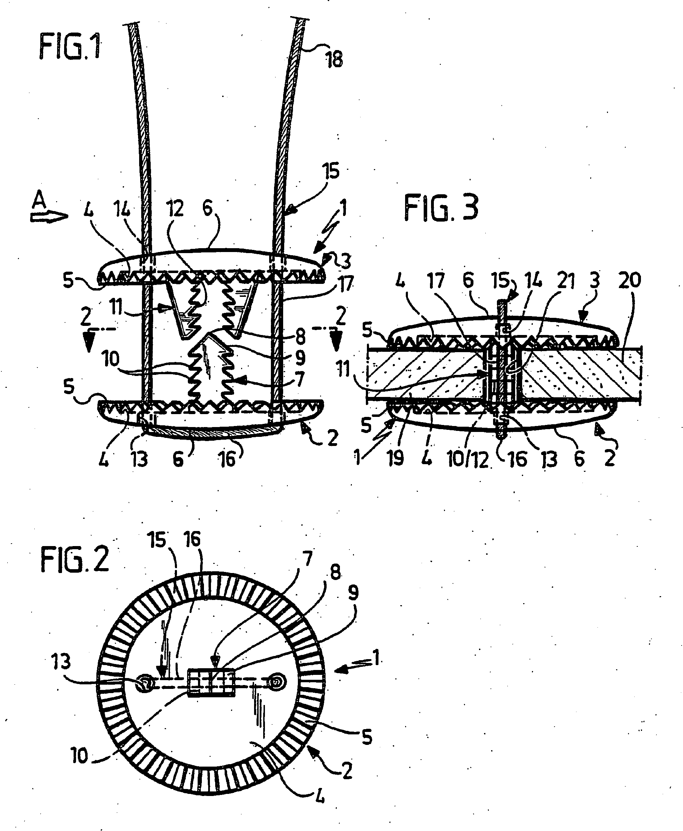

Das in den Figuren 1 bis 3 dargestellte Implantat 1, welches nicht unter die Ansprüche fällt, umfaßt zwei kreisrunde, tellerförmige Anlageelemente 2, 3, die an den einander zugewandten, ebenen Innenseiten 4 einen gezackten Randbereich 5 und eine leicht nach außen gewölbte Außenseite 6 aufweisen.

Das innere Anlageelement 2 trägt einen zentralen, zum äußeren Anlageelement 3 hin weisenden Rastvorsprung 7, der eine Spitze 8 mit schrägen Aufgleitflächen 9 aufweist. Dieser Rastvorsprung 7 hat einen rechteckigen, langgestreckten Querschnitt, und die Schmalseiten tragen eine Anzahl von Rasten 10, während die Breitseiten glatt ausgebildet sind.

Das äußere Anlageelement 3 trägt zwei zum inneren Anlageelement 3 hin weisende, im Abstand zueinander angeordnete Rastvorsprünge 11, die an den einander zugewandten Schmalseiten ebenfalls mit einer Anzahl von Rasten 12 versehen sind. Diese Schmalseiten mit den Rasten 12 haben einen Abstand voneinander, der der Breite des Rastvorsprunges 7 am inneren Anlageelement 2 entspricht. Wenn die beiden Anlageelemente 2, 3 einander angenähert werden, gleiten die Rastvorsprünge 11 mit ihren freien Enden an den schrägen Aufgleitflächen 9 des Rastvorsprunges 7 auf und werden geringfügig elastisch auseinandergebogen, so daß die Rasten 12 an den Rasten 10 vorbeigleiten können. Die Formgebung der Rasten 10 und 12 ist dabei so gewählt, daß zwar eine Annäherung der Anlageelemente 2 und 3 möglich ist, jedoch nach dem Eingreifen der Rasten 10 und 12 keine Entfernung mehr der beiden Anlageelemente 2, 3 voneinander.

Auch die Rastvorsprünge 11 sind im Querschnitt rechteckförmig ausgebildet und sind ebenso wie der Rastvorsprung 7 mit Schmalseiten versehen, die die Rasten 12 tragen, und mit rastenfreien Breitseiten. Die Rastvorsprünge 7 und 11 erstrecken sich somit im wesentlichen in einer Ebene, die parallel zu den Breitseiten der Rastvorsprünge 7 und 11 verläuft, quer dazu haben sie nur eine sehr geringe Ausdehnung, wie dies aus der Darstellung der Figur 3 deutlich wird.

In dieser von den Rastvorsprüngen 7 und 11 aufgespannten Ebene befinden sich sowohl im inneren Anlageelement 2 als auch im äußeren Anlageelement 3 jeweils zwei die Anlageelemente durchsetzende Öffnungen 13 beziehungsweise 14, die voneinander einen Abstand einhalten, der mindestens dem Radius der Anlageelemente 2, 3 entspricht und die symmetrisch zu beiden Seiten der Rastvorsprünge 7 beziehungsweise 11 angeordnet sind. Durch diese Öffnungen 13, 14 ist ein fadenförmiges Spannelement 15 so hindurchgeführt, daß ein Mittelabschnitt 16 zwischen den Öffnungen 13 des inneren Anlageelementes 2 an der Außenseite 6 des Anlageelementes 2 anliegt, während zwei Verbindungsabschnitte 17 parallel zum Rastvorsprung 7 von je einer Öffnung 13 im inneren Anlageelement 2 zu je einer Öffnung 14 im äußeren Anlageelement 3 verlaufen, die beiden Enden 18 des Spannelementes 15 führen durch die Öffnungen 14 hindurch und setzen sich über die Außenseite 6 des äußeren Anlageelementes 3 hin fort (Figur 1).

Die beiden Anlageelemente 2 und 3 bestehen vorzugsweise aus einem körperverträglichen Kunststoffmaterial, die Rastvorsprünge 7 beziehungsweise 10 sind einstückig mit den Anlageelementen 2 beziehungsweise 3 ausgebildet. Das fadenförmige Spannelement 15 kann ein üblicher chirurgischer Faden sein, günstig ist es, wenn dieser aus einem resorbierbaren Material besteht, das nach Einsetzen im Körper allmählich abgebaut wird.

Das beschriebene Implantat 1 dient der Festlegung von zwei Knochenplatten 19, 20 des Schädelknochens, die unter Ausbildung eines Trennspaltes 21 unmittelbar nebeneinander festgelegt werden sollen. Der Trennspalt 21 ist üblicherweise ein Sägespalt, der entsteht, wenn eine Knochenplatten aus dem Schädelknochen herausgesägt wird. Somit ist üblicherweise eine der beiden Knochenplatten die feste Knochenplatte des Schädels, die andere Knochenplatte eine herausgesägte Schädelkalotte, die einen Zugang zum Gehirn freigibt. Zur Festlegung der beiden Knochenplatten aneinander wird zunächst das Implantat 1 in der aus Figur 1 ersichtlichen Weise so an eine Knochenplatte herangeführt, daß das innere Anlageelement 2 an der Innenseite der Knochenplatte zur Anlage kommt. Die andere Knochenplatte wird dann seitlich in den Zwischenraum zwischen die beiden noch voneinander entfernten Anlageelemente 2, 3 eingeführt, und das innere Anlageelement 2 wird dann mit Hilfe des Spannelementes 15 gegen die Innenseite der beiden Knochenplatten 19, 20 gespannt. Bei festgehaltenem inneren Anlageelement 2 wird das äußere Anlageelement 3 gegen das innere Anlageelement 2 verschoben, bis die Rasten 10 und 12 der beiden Rastvorsprünge 7 beziehungsweise 11 in Eingriff kommen. Dieses Vorschieben des äußeren Anlageelementes 3 kann entweder mit der Hand oder einem Instrument erfolgen, wobei das innere Anlageelement 2 mit Hilfe des Spannelementes 15 in seiner Anlage an der Innenseite der Knochenplatten gehalten wird, oder aber dadurch, daß die beiden Enden des Spannelementes 15 gegeneinander gezogen werden, beispielsweise auch mittels eines an sich bekannten Spannknotens, durch den eine Verkürzung der von dem Spannelement 15 gebildeten, durch die Öffnungen 13 und 14 hindurchlaufenden Schlaufe möglich ist, nicht jedoch eine Verlängerung.

Die Rastvorsprünge 7 und 10 liegen in derselben Ebene, in der auch die Verbindungsabschnitte 17 des Spannelementes 15 verlaufen, alle Teile weisen quer zu dieser Ebene eine sehr geringe Ausdehnung auf, so daß diese Teile ohne weiteres auch in einen sehr schmalen Trennspalt 21 hineinpassen, wie dies aus der Darstellung der Figur 3 deutlich wird. Gleichzeitig orientieren die Rastvorsprünge 7 und 11 sowie die Mittelabschnitte 17 des Spannelementes 15 die beiden Anlageelemente 2, 3 so, daß diese Ebene parallel zum Trennspalt 21 verläuft.

Wenn die beiden Anlageelemente 2, 3 in dieser Weise gegeneinander gespannt sind, werden sie in dieser Anlagestellung, die in Figur 3 dargestellt ist, durch den Eingriff der Rasten 10 und 12 sicher gehalten. Das Spannelement 15 kann jetzt entweder entfernt werden, indem es einfach aus den Öffnungen 13, 14 herausgezogen wird, oder es wird als zusätzliche Sicherung belassen, wobei es günstig ist, die beiden Enden 18 miteinander zu verknoten.

Bei dem in den Figuren 1 bis 3 dargestellten Implantat ragen die durch die Rastvorsprünge 7 und 11 gebildeten Verbindungsmittel der beiden Anlageelemente 2, 3 nicht durch diese hindurch, sondern sie erstrecken sich nur in den Raum des Trennspaltes 21 hinein.

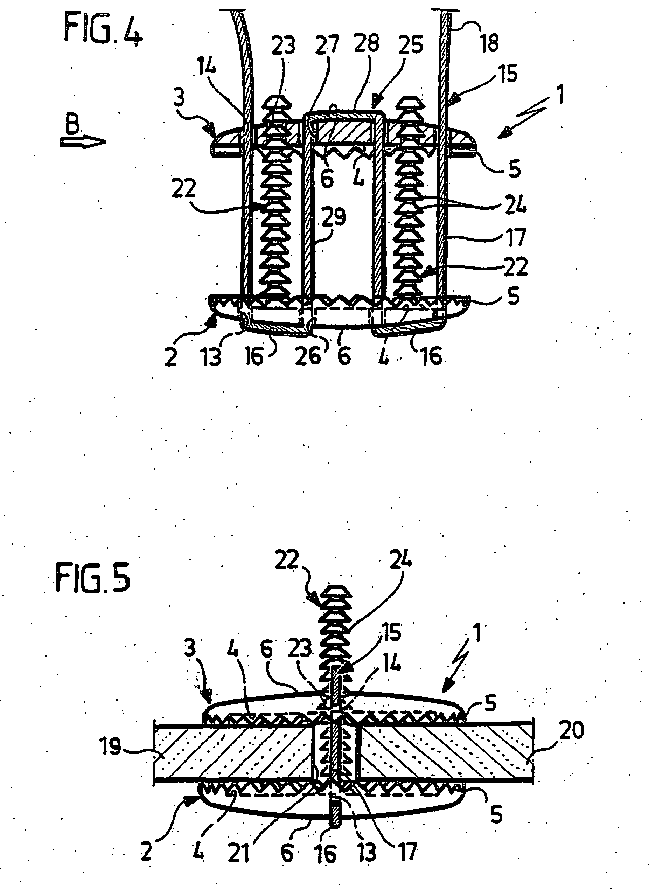

Dies ist bei dem in den Figuren 4 und 5 dargestellten Ausführungsbeispiel eines Implantates, das ähnlich aufgebaut ist und bei dem einander entsprechende Teile dieselben Bezugszeichen tragen, nicht der Fall. Bei dem Implantat der Figuren 4 und 5 trägt das innere Anlageelement 2 im Abstand zueinander und symmetrisch zum Mittelpunkt zwei parallel verlaufende Verbindungsstifte 22, die durch Durchbrechungen 23 im äußeren Anlageelement 3 hindurchragen. Die Verbindungsstifte 22 tragen umlaufende Rastrippen 24, die einseitig abgeschrägt sind und die in einer Richtung durch die Durchbrechungen 23 hindurchgeschoben werden können, in entgegengesetzter Richtung aber die Ränder der Durchbrechungen 23 derart rastend hintergreifen, daß ein Auseinanderziehen der Anlageelemente 2 und 3 nicht mehr möglich ist. Die Ränder der Durchbrechungen 23 sind somit Rastvorsprünge, die mit den Rastrippen 24 zusammenwirken.

Die Verbindungsstifte 22 sind in der Ebene angeordnet, die durch die Öffnungen 13 und 14 für das Spannelement 15 definiert ist, und sie haben einen sehr geringen Durchmesser, so daß die Verbindungsstifte 22 quer zu dieser Ebene nur eine sehr geringe Abmessung aufweisen, dadurch kann auch dieses Implantat 1 mit dem Spannelement 15 und den beiden Verbindungsstiften 22 in einem sehr schmalen Trennspalt 21 Platz finden.

Bei dem Ausführungsbeispiel der Figuren 4 und 5 ist außerdem das Spannelement 15 in seinem mittleren Bereich noch in Form einer zusätzlichen Schlaufe 25 abweichend vom Verlauf des Implantates der Figuren 1 bis 3 geführt. Diese Schlaufe 25 tritt nämlich durch zwei zwischen den Öffnungen 13 am inneren Anlageelement 2 liegenden Öffnungen 26 und durch zwei zwischen den Öffnungen 14 am äußeren Anlageelement 3 angeordneten Öffnungen 27 hindurch, so daß das Mittelteil 28 dieser Schlaufe 25 an der Außenseite 6 des äußeren Anlageelementes 3 anliegt. Die Öffnungen 26 und 27 befinden sich dabei dichter am Zentrum der Anlageelemente 2 beziehungsweise 3 als die Durchbrechungen 23, so daß zwischen den beiden Verbindungsstiften 22 zusätzlich zu den Verbindungsabschnitten 17 des Spannelementes 15 noch zwei weitere Verbindungselemente 29 verlaufen, diese bilden die Schenkel der Schlaufe 25 aus und liegen in derselben Ebene wie die Verbindungsabschnitte 17 und die Verbindungsstifte 22. Dadurch ergibt sich eine flaschenzugartige Führung des Spannelementes 15, so daß beim Spannen desselben der Verschiebeweg der Anlageelemente 2, 3 gegenüber dem Spannweg des Spannelementes 15 herabgesetzt wird.

Diese Führung des Spannelementes 15 ist besonders vorteilhaft bei einer Ausgestaltung mit zwei im Abstand zueinander angeordneten Verbindungsmitteln, wie es die Verbindungsstifte 22 ausbilden, könnte aber ohne weiteres auch bei anders aufgebauten Anlageelementen 2, 3 Verwendung finden, bei denen andere Verbindungsmittel vorgesehen sind.

Auch bei dem Ausführungsbeispiel der Figuren 4 und 5 ist es günstig, wenn die Verbindungsstifte 22 einstückig mit den Anlageelementen 2, 3 ausgebildet sind, wobei diese vorzugsweise aus einem körperverträglichen Kunststoff bestehen. Auch hier kann das Spannelement 15 aus einem resorbierbaren Werkstoff hergestellt sein.

## Patentansprüche

1. Implantat zur Fixierung benachbarter eine Innenseite und eine Außenseite aufweisender Knochenplatten (19, 20) des Schädelknochens, mit einem einen Trennspalt (21) zwischen den Knochenplatten (19, 20) überdeckenden inneren Anlageelement (2), einem den Trennspalt (21) überdeckenden äußeren Anlageelement (3) und mit den Trennspalt (21) durchsetzenden Verbindungsmitteln (15),
**dadurch gekennzeichnet, daß** die beiden Anlageelemente (2, 3) durch ein fadenförmiges Spannelement (15) verbunden sind, welches durch das äußere Anlageelement (3) verschieblich hindurchgeführt ist und bei Zug das innere Anlageelement (2) gegen das äußere Anlageelement (3) verschiebt, daß das Spannelement (15) durch zwei Öffnungen (13) im inneren Anlageelement (2) hindurchgeführt ist und im Bereich zwischen den Öffnungen (13) auf der dem äußeren Anlageelement (3) abgewandten Außenseite (6) des inneren Anlageelementes (2) verläuft und daß das Spannelement (15) von der Außenseite (6) des inneren Anlageelementes (2) durch dieses hindurch in einer Schlaufe (25) geführt ist, die durch zwei im Abstand zueinander angeordnete Öffnungen (27) in dem äußeren Anlageelement (3) verläuft, so daß die Schlaufe (25) zwischen diesen Öffnungen (27) an der vom Inneren Anlageelement (2) abgewandten Außenseite (6) des äußeren Anlageelementes (3) anliegt, wobei die Schlaufe (25) durch zwei im Abstand zueinander angeordnete Öffnungen (26) am inneren Anlageelement (2) hindurchläuft.

2. Implantat nach Anspruch 1, **dadurch gekennzeichnet, daß** beide Enden (18) des Spannelementes (15) durch je eine Öffnung (14) des äußeren Anlageelementes (3) hindurchgeführt sind.

3. Implantat nach einem der vorangehenden Ansprüche, **dadurch gekennzeichnet, daß** die beiden Öffnungen (13) im inneren Anlageelement (2) einen Abstand voneinander haben, der mindestens der halben Ausdehnung des inneren Anlageelements (2) in ihrer Verbindungsrichtung entspricht.

4. Implantat nach einem der vorangehenden Ansprüche, **dadurch gekennzeichnet, daß** Verbindungsmittel (7, 11; 22) beim Annähern der Anlageelemente (2, 3) diese durch eine Rast- oder Klemmverbindung derart miteinander verbinden, daß sie nicht mehr voneinander lösbar sind.

5. Implantat nach Anspruch 4, **dadurch gekennzeichnet, daß** die Verbindungsmittel (7, 11; 22) zwischen den beiden zwischen den Anlageelementen (2, 3) verlaufenden Abschnitten (17) des Spannelementes (15) angeordnet sind.

6. Implantat nach Anspruch 5, **dadurch gekennzeichnet, daß** die Verbindungsmittel (7, 11; 22) in einer Richtung quer zu der von den zwischen den beiden Anlageelementen (2, 3) verlaufenden Abschnitten (17) des Spannelementes (15) aufgespannten Ebene eine geringe Ausdehnung aufweisen, um so den Durchgang durch den Trennspalt (21) zu ermöglichen.

7. Implantat nach einem der vorangehenden Ansprüche, **dadurch gekennzeichnet, daß** die Öffnungen (27) der Schlaufe (25) und die Öffnungen (14) der Enden (18) des Spannelementes (15) am äußeren Anlageelement (3) auf einer Geraden liegen.

8. Implantat nach einem der vorangehenden Ansprüche, **dadurch gekennzeichnet, daß** die Öffnungen (27) der Schlaufe (25) und die Öffnungen (14) der Enden (18) des Spannelementes (15) äquidistant zueinander angeordnet sind.

9. Implantat nach einem der vorangehenden Ansprüche, **dadurch gekennzeichnet, daß** die Öffnungen (26) der Schlaufe (25) und die Öffnungen (13) der Enden (18) des Spannelementes (15) am inneren Anlageelement (2) auf einer Geraden liegen.

10. Implantat nach einem der vorangehenden Ansprüche, **dadurch gekennzeichnet, daß** die Öffnungen (26) der Schlaufe (25) und die Öffnungen (13) der Enden (18) des Spannelementes (15) am inneren Anlageelement (2) äquidistant zueinander angeordnet sind.

11. Implantat nach einem der vorangehenden Ansprüche, **dadurch gekennzeichnet, daß** die zwischen den Anlageelementen (2, 3) verlaufenden Abschnitte (17) des Spannelementes (15), die Schlaufe (25) und Verbindungsmittel (7, 11; 22) in einer Ebene angeordnet sind.

12. Implantat nach einem der voranstehenden Ansprüche, **dadurch gekennzeichnet, daß** das Spannelement (15) aus einem im Körper resorbierbaren Material besteht.

13. Implantat nach einem der voranstehenden Ansprüche, **dadurch gekennzeichnet, daß** Verbindungsmittel (7, 11) im Bereich zwischen den Anlageelementen (2, 3) angeordnet sind und nicht durch diese hindurch nach außen ragen.

14. Implantat nach Anspruch 13, **dadurch gekennzeichnet, daß** an beiden Anlageelementen (2, 3) an deren dem anderen Anlageelement (3, 2) zugewandten Innenseite (4) Rastvorsprünge (10, 12) angeordnet sind, die sich bei Annäherung der Anlageelemente (2, 3) rastend hintergreifen.

15. Implantat nach Anspruch 14, **dadurch gekennzeichnet, daß** an einem Anlageelement (3) nebeneinander zwei Rastelemente (11) angeordnet sind, zwischen die ein Rastelement (7) am anderen Anlageelement (2) bei Annäherung der Anlageelemente (2, 3) rastend eingreift.

16. Implantat nach Anspruch 14 oder 15, **dadurch gekennzeichnet, daß** min-destens eines der Rastelemente (7, 11) eine Vielzahl von Rastvorsprüngen (10, 12) trägt.

17. Implantat nach einem der voranstehenden Ansprüche, **dadurch gekennzeichnet, daß** nebeneinander an den Anlageelementen (2, 3) mindestens zwei Verbindungsmittel (22, 23) angeordnet sind, die quer zu der von ihnen definierten Ebene eine geringe Ausdehnung aufweisen.

18. Implantat nach Anspruch 17, **dadurch gekennzeichnet, daß** die Verbindungsmittel Rast- oder Klemmstifte (22) umfassen, die am inneren Anlageelement (2) festgelegt sind und Rast- oder Klemmöffnungen (23) in dem äußeren Anlageelement (3) durchsetzen.

19. Implantat nach Anspruch 18, **dadurch gekennzeichnet, daß** die Rast-oder Klemmöffnungen (23) Rastvorsprünge tragen, die mit Rastvorsprüngen (24) an den Rast- oder Klemmstiften (22) zusammenwirken.

20. Implantat nach Anspruch 19, **dadurch gekennzeichnet, daß** die Rast-oder Klemmöffnungen (23) elastisch verformbare Klemmglieder aufweisen, die mit der Oberfläche der Rast- oder Klemmstifte (22) klemmend zusammenwirken.

21. Implantat nach einem der voranstehenden Ansprüche, **dadurch gekennzeichnet, daß** die Anlageelemente (2, 3) und Verbindungsmittel (7, 11; 22) einstückig aus Kunststoff bestehen.

## Claims

1. Implant for fixing neighbouring bone plates (19, 20), having an inner surface and an outer surface, of the cranial bone, the implant comprising an inner bearing element (2) which covers a spacing gap (21) between the bone plates (19,20), an outer bearing element (3) which covers the spacing gap (21), and connecting means (15) which extend through the spacing gap (21), **characterized in that** the two bearing elements (2, 3) are connected by means of a thread-like tensioning element (15) which is passed through the outer bearing element (3) in displaceable manner and, when tensioned, moves the inner bearing element (2) towards the outer bearing element (3), **in that** the tensioning element (15) is passed through two openings (13) in the inner bearing element (2) and extends over the outer surface (6) of the inner bearing element (2) which is remote from the outer bearing element (3) in the area between the openings (13), and **in that** the tensioning element (15) is fed through the inner bearing element (2) from the outer surface (6) thereof in the form of a loop (25) which extends through two mutually spaced openings (27) in the outer bearing element (3) so that between these openings (27) the loop (25) rests on the outer surface (6) of the outer bearing element (3) which is remote from the inner bearing element (2), with the loop (25) extending through two mutually spaced openings (26) in the inner bearing element (2).

2. Implant in accordance with claim 1, **characterized in that** the two ends (18) of the tensioning element (15) are passed through a respective opening (14) in the outer bearing element (3).

3. Implant in accordance with any one of the preceding claims, **characterized in that** the two openings (13) in the inner bearing element (2) are spaced from one another by a distance which corresponds to at least half the elongation of the inner bearing element (2) in the direction of interconnection.

4. Implant in accordance with any one of the preceding claims, **characterized in that** when the bearing elements (2, 3) approach one another, connecting means (7, 11; 22) connect the bearing elements together by means of a latching or a clamping connection in such a manner that they are no longer able to be moved apart.

5. Implant in accordance with claim 4, **characterized in that** the connecting means (7, 11; 22) are arranged between the two sections (17) of the tensioning element (15) extending between the bearing elements (2, 3).

6. Implant in accordance with claim 5, **characterized in that** the dimensions of the connecting means (7, 11; 22) in a direction transverse to the plane spanned by the sections (17) of the tensioning element (15) extending between the two bearing elements (2, 3) are small so as to enable the passage thereof through the spacing gap (21).

7. Implant in accordance with any one of the preceding claims, **characterized in that** the openings (27) for the loop (25) and the openings (14) for the ends (18) of the tensioning element (15) in the outer bearing element (3) lie along a straight line.

8. Implant in accordance with any one of the preceding claims, **characterized in that** the openings (27) for the loop (25) and the openings (14) for the ends (18) of the tensioning element (15) are mutually equidistant.

9. Implant in accordance with any one of the preceding claims, **characterized in that** the openings (26) for the loop (25) and the openings (13) for the ends (18) of the tensioning element (15) in the inner bearing element (2) lie along a straight line.

10. Implant in accordance with any one of the preceding claims, **characterized in that** the openings (26) for the loop (25) and the openings (13) for the ends (18) of the tensioning element (15) in the inner bearing element (2) are mutually equidistant.

11. Implant in accordance with any one of the preceding claims, **characterized in that** the sections (17) of the tensioning element (15) extending between the bearing elements (2, 3), the loop (25) and the connecting means (7, 11; 22) are arranged in one plane.

12. Implant in accordance with any one of the preceding claims, **characterized in that** the tensioning element (15) consists of a material that is absorbable in the body.

13. Implant in accordance with any one of the preceding claims, **characterized in that** the connecting means (7, 11) are arranged in the area between the bearing elements (2, 3) and do not project outwardly therethrough.

14. Implant in accordance with claim 13, **characterized in that** there are arranged on both bearing elements (2, 3) on the inner surface (4) thereof facing the other bearing element (3, 2) latching projections (10, 12) which engage behind one another in latching manner when the bearing elements (2, 3) approach one another.

15. Implant in accordance with claim 14, **characterized in that** there are arranged adjacent to each other on a bearing element (3) two latching elements (11) between which a latching element (7) on the other bearing element (2) engages in latching manner when the bearing elements (2, 3) approach one another.

16. Implant in accordance with claim 14 or 15, **characterized in that** at least one of the latching elements (7, 11) carries a plurality of latching projections (10, 12).

17. Implant in accordance with any one of the preceding claims, **characterized in that** there are arranged adjacent to each other on the bearing elements (2, 3) at least two connecting means (22, 23) which have small dimensions in a direction transverse to the plane defined by the connecting means.

18. Implant in accordance with claim 17, **characterized in that** the connecting means comprise latching or clamping pins (22) which are fixed to the inner bearing element (2) and penetrate through latching or clamping openings (23) in the outer bearing element (3).

19. Implant in accordance with claim 18, **characterized in that** the latching or clamping openings (23) carry latching projections which co-operate with latching projections (24) on the latching or clamping pins (22).

20. Implant in accordance with claim 19, **characterized in that** the latching or clamping openings (23) comprise resiliently deformable clamping members which co-operate with the surface of the latching or clamping pins (22).

21. Implant in accordance with any one of the preceding claims, **characterized in that** the bearing elements (2, 3) and the connecting means (7, 11; 22) are formed in one piece from a synthetic material.

## Revendications

1. Implant destiné à la fixation de plaques d'ostéosynthèse (19, 20) de l'os crânien adjacentes, munies d'une face intérieure et d'une face extérieure, comportant un élément d'appui intérieur (2), couvrant une fente de séparation (21) entre les plaques d'ostéosynthèse (19, 20), un élément d'appui extérieur (3), couvrant la fente de séparation (21), et des moyens de liaison (15) traversant la fente de séparation (21),
**caractérisé en ce que** les deux éléments d'appui (2, 3) sont reliés par un élément de serrage (15) en forme de fil, qui est guidé de manière mobile à travers l'élément d'appui extérieur (3) et, en cas de traction, déplace l'élément d'appui intérieur (2) contre l'élément d'appui extérieur (3), **en ce que** l'élément de serrage (15) est guidé à travers deux orifices (13) dans l'élément d'appui intérieur (2) et s'étend dans la zone entre les orifices (13) sur la face extérieure (6) de l'élément d'appui intérieur (2), détournée de l'élément d'appui extérieur (3), et **en ce que** l'élément de serrage (15) est guidé depuis la face extérieure (6) de l'élément d'appui intérieur (2) à travers celle-ci en formant une boucle (25) qui passe à travers deux orifices (27), écartés l'un de l'autre, dans l'élément d'appui extérieur (3), de telle sorte que la boucle (25), entre lesdits orifices (27) est en appui contre la face extérieure (6) de l'élément d'appui extérieur (3), détournée de l'élément d'appui intérieur (2), la boucle (25) passant à travers deux orifices (26) écartés l'un de l'autre dans l'élément d'appui intérieur (2).

2. Implant selon la revendication 1, **caractérisé en ce que** les deux extrémités (18) de l'élément de serrage (15) sont guidées chacune à travers un orifice (14) de l'élément d'appui extérieur (3).

3. Implant selon l'une quelconque des revendications précédentes, **caractérisé en ce que** les deux orifices (13) dans l'élément d'appui intérieur (2) sont situés l'un de l'autre à une distance qui correspond au moins à la moitié de l'extension de l'élément d'appui intérieur (2) dans sa direction d'assemblage.

4. Implant selon l'une quelconque des revendications précédentes, **caractérisé en ce que** des moyens d'assemblage (7, 11 ; 22), lors du rapprochement des éléments d'appui (2, 3), relient ceux-ci l'un à l'autre par un assemblage bloqué ou serré, de telle sorte qu'ils ne peuvent plus être désolidarisés l'un de l'autre.

5. Implant selon la revendication 4, **caractérisé en ce que** les moyens d'assemblage (7, 11 ; 22) sont disposés entre les deux parties (17) de l'élément de serrage (15), qui s'étendent entre les éléments d'appui (2, 3).

6. Implant selon la revendication 5, **caractérisé en ce que** les moyens d'assemblage (7, 11 ; 22) ont une faible dimension dans une direction transversale au plan déployé par les parties (17) de l'élément de serrage (15), qui s'étendent entre les éléments d'appui (2, 3), afin de permettre ainsi le passage à travers la fente de séparation (21).

7. Implant selon l'une quelconque des revendications précédentes, **caractérisé en ce que** les orifices (27) de la boucle (25) et les orifices (14) des extrémités (18) de l'élément de serrage (15) sur l'élément d'appui extérieur (3) sont situés sur une droite.

8. Implant selon l'une quelconque des revendications précédentes, **caractérisé en ce que** les orifices (27) de la boucle (25) et les orifices (14) des extrémités (18) de l'élément de serrage (15) sont situés à équidistance les uns des autres.

9. Implant selon l'une quelconque des revendications précédentes, **caractérisé en ce que** les orifices (26) de la boucle (25) et les orifices (13) des extrémités (18) de l'élément de serrage (15) sur l'élément d'appui intérieur (2) sont situés sur une droite.

10. Implant selon l'une quelconque des revendications précédentes, **caractérisé en ce que** les orifices (26) de la boucle (25) et les orifices (13) des extrémités (18) de l'élément de serrage (15) sur l'élément d'appui intérieur (2) sont situés à équidistance les uns des autres.

11. Implant selon l'une quelconque des revendications précédentes, **caractérisé en ce que** les parties (17) de l'élément de serrage (15), qui s'étendent entre les éléments d'appui (2, 3), la boucle (25) et les moyens d'assemblage (7, 11 ; 22) sont disposés dans un plan.

12. Implant selon l'une quelconque des revendications précédentes, **caractérisé en ce que** l'élément de serrage (15) est réalisé dans un matériau résorbable dans le corps.

13. Implant selon l'une quelconque des revendications précédentes, **caractérisé en ce que** les moyens d'assemblage (7, 11) sont disposés dans la zone entre les éléments d'appui (2, 3) et ne s'avancent pas vers l'extérieur à travers ceux-ci.

14. Implant selon la revendication 13, **caractérisé en ce que** sur les deux éléments d'appui (2, 3), sur leur face intérieure (4) tournée vers l'autre élément d'appui (3, 2), sont disposées des saillies de blocage (10, 12), qui se bloquent l'une derrière l'autre au moment du rapprochement des éléments d'appui (2, 3).

15. Implant selon la revendication 14, **caractérisé en ce que** sur un élément d'appui (3) sont disposés côte à côte deux éléments de blocage (11), entre lesquels s'engage de manière bloquée un élément de blocage (7) sur l'autre élément d'appui (2) au moment du rapprochement des éléments d'appui (2, 3).

16. Implant selon la revendication 14 ou 15, **caractérisé en ce qu'**au moins un des éléments de blocage (7, 11) comporte une pluralité de saillies de blocage (10, 12).

17. Implant selon l'une quelconque des revendications précédentes, **caractérisé en ce que** deux moyens d'assemblage (22, 23) au moins sont disposés côte à côte sur les éléments d'appui (2, 3) et ont une faible dimension transversalement au plan qu'ils définissent.

18. Implant selon la revendication 17, **caractérisé en ce que** les moyens d'assemblage comportent des tétons de blocage ou serrage (22), qui sont fixés sur l'élément d'appui intérieur (2), et des orifices de blocage ou serrage (23) qui traversent l'élément d'appui extérieur (3).

19. Implant selon la revendication 18, **caractérisé en ce que** les orifices de blocage ou serrage (23) portent des saillies de blocage, qui coopèrent avec des saillies de blocage (24) sur les tétons de blocage ou serrage (22).

20. Implant selon la revendication 19, **caractérisé en ce que** les orifices de blocage ou serrage (23) comportent des organes de serrage élastiquement déformables, qui coopèrent par serrage avec la surface des tétons de blocage ou serrage (22).

21. Implant selon l'une quelconque des revendications précédentes, **caractérisé en ce que** les éléments d'appui (2, 3) et les moyens d'assemblage (7, 11 ; 22) sont réalisés d'un seul tenant en matière plastique.
